# EUROPÄISCHE PATENTSCHRIFT

(11) **EP 1 464 659 B1**
(45) Veröffentlichungstag und Bekanntmachung des Hinweises auf die Patenterteilung: **06.10.2010**
(21) Anmeldenummer: 04007372.8
(22) Anmeldetag: 26.03.2004
(51) Int. Cl.: C08F 20/58, C08F 6/12, A61K 8/81

(54) **Verfahren zur Herstellung von stabilen Polymer-Konzentraten**
Process for preparing stable polymer concentrates
Procédé de préparation de concentrés stables de polymères

(30) Priorität: 03.04.2003 DE 10315183
(43) Veröffentlichungstag der Anmeldung: 06.10.2004
(73) Patentinhaber: Clariant Produkte (Deutschland) GmbH, 65929 Frankfurt am Main (DE)
(72) Erfinder: Löffler, Matthias, Dr., 65527 Niedernhausen (DE); Morschhäuser, Roman, Dr., 55122 Mainz (DE)

(56) Entgegenhaltungen:
- DE-A- 19 625 810

## Beschreibung

Die vorliegende Erfindung betrifft ein Verfahren zur Herstellung von Konzentraten aus Copolymeren auf Basis von Acryloyldimethyltaurinsäure bzw. deren Salzen.

Die in EP 816 403 beschriebenen Polymere auf Basis von Acryloyldimethyltaurinsäure bzw. deren Salzen, bevorzugt hergestellt durch Fällungspolymerisation, decken breite anwendungstechnische Eigenschaften ab und können als Verdicker, Konsistenzgeber, Emulgator, Dispergator, Gleitmittel, Conditioner und/oder Stabilisator in kosmetischen, dermatologischen und pharmazeutischen Mitteln eingesetzt werden.

Die bevorzugt durch Fällungspolymerisation hergestellten Copolymere auf Basis von Acryloyldimethyltaurinsäure bzw. deren Salzen entsprechend dem Stand der Technik sind pulverförmige Substanzen mit dadurch resultierenden anwendungstechnischen Nachteilen. Pulverförmige Substanzen bergen prinzipiell Staubexplosionsgefahr, ferner ist die Lagerstabilität der Pulver durch Hygroskopie beeinträchtigt.

Zur Verarbeitung bzw. Verwendung der pulverförmigen Produkte ist der Lösevorgang (bevorzugt werden die Polymere in wässrige Medien eingearbeitet) meistens sehr zeitaufwendig. Der Lösevorgang der pulverförmigen Produkte kann, je nach Ansatzgröße, eine Stunde und mehr betragen. Zudem wird häufig eine unvollständige Lösung/Aufquellung der pulverförmigen Produkte beobachtet, was zu einer Qualitäts- und Stabilitätsverminderung der Endformulierung führt (Klumpenund Quaddelbildung). Des weiteren sind bei der Verarbeitung bzw. Verwendung der pulverförmigen Produkte im allgemeinen besondere Rühr- und Dispergiervorrichtungen erforderlich, um die Polymere auf Basis von Acryloyldimethyltaurinsäure bzw. deren Salzen in den Mitteln zu lösen, bzw. zu suspendieren.

Aufgabe der vorliegenden Erfindung war die Entwicklung eines Verfahrens zur Herstellung von Polymerkonzentraten, enthaltend Polymere auf Basis von Acryloyldimethyltaurinsäure bzw. deren Salzen. Die Polymerkonzentrate sollten bei Vorliegen in hochkonzentrierter flüssiger oder flüssig-disperser Form, d.h. bei Auftreten eines möglichst hohen Polymeranteils, eine niedrige Viskosität bei gleichzeitig hoher Stabilität der Lösung bzw. Dispersion aufweisen.

Überraschenderweise wurde gefunden, dass lagerstabile und thermostabile Konzentrate in flüssiger oder flüssig-disperser Form aus nachfolgend beschriebenen Copolymeren enthaltend Acryloyldimethyltaurinsäure bzw. deren Salze hergestellt werden können, wenn man im Anschluss an die Polymerisationsreaktion ein Lösungsmittel oder Lösungsmittelgemisch zusetzt, dessen Siedepunkt höher ist, als der Siedepunkt des zur Polymerisation eingesetzten Polymerisationsmediums, Lösungsmittels bzw. Lösungsmittelgemisches und anschließend das niedriger siedende Polymerisationsmedium, Lösungsmittel bzw. Lösungsmittelgemisch, gegebenenfalls bei einem Druck, der im Vergleich zu Atmosphärendruck erniedrigt ist, und gegebenenfalls bei einer Temperatur, die im Vergleich zu Raumtemperatur (25°C) erhöht ist, entfernt.

Gegenstand der Erfindung ist ein Verfahren zur Herstellung von Konzentraten in flüssiger oder flüssig-disperser Form, enthaltend
I) 5 bis 80 Gew.-%, bevorzugt 10 bis 80 Gew.-%, besonders bevorzugt 20 bis 60 Gew.-%, insbesondere bevorzugt 30 bis 40 Gew.-% eines Polymers, das in statistischer Verteilung zu 90 bis 99,99 Gew.-% aus Monomeren der allgemeinen Formel (1)

   H₂C=CHCONHC(CH₃)₂CH₂SO₃X (1)

   worin X für ein Kation oder ein Gemisch von Kationen steht und X nicht zu mehr als 10 Mol-% aus Protonen besteht, und
   zu 0,01 bis 10 Gew.-% aus Monomeren mit mindestens zwei olefinischen Doppelbindungen hervorgegangen ist,
II) 20 bis 95 Gew.-%, bevorzugt 20 bis 90 Gew.-%, besonders bevorzugt 40 bis 80 Gew.-%, insbesondere bevorzugt 60 bis 70 Gew.-% eines oder mehrerer Emulgatoren und/oder eines Lösungsmittels oder Lösungsmittelgemischs und
III) 0 bis 30 Gew.-%, bevorzugt 0 bis 10 Gew.-%, besonders bevorzugt 0 bis 5 Gew.-% Wasser,
dadurch gekennzeichnet, dass das Konzentrat hergestellt wird durch
a) radikalische Polymerisation der Monomere gemäß Formel (1) in Gegenwart der Monomere mit mindestens zwei olefinischen Doppelbindungen, vorzugsweise durch Lösungspolymerisation, Gelpolymerisation, nach einem Emulsionsverfahren, Fällungsverfahren, Hochdruckverfahren oder Suspensionsverfahren, in einem Polymerisationsmedium, das sich bezüglich radikalischer Polymerisationsreaktionen weitestgehend inert verhält und die Bildung hoher Molekulargewichte zulässt, bevorzugt Wasser und niedere, tertiäre Alkohole oder Kohlenwasserstoffe mit 3 bis 30 C-Atomen, besonders bevorzugt tert.-Butanol,
b) dem Gemisch aus Polymer und Polymerisationsmedium ein höhersiedendes Lösungsmittel bzw. Lösungsmittelgemisch und/oder ein oder mehrere Emulgatoren und gegebenenfalls Wasser zugesetzt wird, wobei der Siedepunkt des höhersiedenden Lösungsmittels bzw. Lösungsmittelgemisches mindestens 10°C höher liegt als der des zur Polymerisation eingesetzten Polymerisationsmediums und
c) das niedriger siedende Polymerisationsmedium, gegebenenfalls bei einem Druck, der im Vergleich zu Atmosphärendruck erniedrigt ist, und gegebenenfalls bei einer Temperatur, die im Vergleich zu Raumtemperatur erhöht ist, entfernt wird.

Bevorzugt enthalten die nach dem erfindungsgemäßen Verfahren hergestellten Konzentrate Polymere, enthaltend 98 bis 99,5 Gew.-% an Einheiten, die aus Monomeren der allgemeinen Formel (1) hervorgegangen sind, und 0,5 bis 2 Gew.-% an vernetzenden Strukturen, die aus Monomeren mit mindestens zwei olefinischen Doppelbindungen hervorgegangen sind.

Das Gegenion X in der Formel (1) steht insbesondere für ein Proton, ein Kation eines Alkalimetalls, ein Äquivalent eines Kations eines Erdalkalimetalls oder für ein Ammoniumion.

Besonders bevorzugte Polymere sind dadurch gekennzeichnet, dass 90 bis 100 Mol-% der Kationen X aus Ammoniumionen und 0 bis 10 Mol-% aus Protonen bestehen.

Die Monomere mit mindestens zwei olefinischen Doppelbindungen sind bevorzugt ausgewählt aus Dipropylglykoldiallylether, Polyglykoldiallylether, Triethylenglykol-divinylether, Hydrochinondiallylether, Tetraallyloxyethan oder andere Allyl- oder Vinylether multifunktioneller Alkohole, Tetraethylenglykoldiacrylat, Triallylamin, Trimethylolpropandiallylether, Methylen-bis-acrylamid, Divinylbenzol oder Trimethylolpropyltri(meth)acrylat. Die Monomere mit mindestens zwei olefinischen Doppelbindungen führen bei der Polymerisation zu vernetzenden Strukturen.

Die nach dem erfindungsgemäßen Verfahren hergestellten Konzentrate enthalten Polymere, die durch radikalische Polymerisation von 90 - 99,99 Gew.-% der Verbindungen der allgemeinen Formel (1) und 0,01 - 10 Gew.-% eines oder mehrerer Vernetzer, die mindestens zwei olefinische Doppelbindungen enthalten, hergestellt werden.

Bevorzugt werden diese Polymere hergestellt durch
a) Lösen oder Dispergieren der Verbindungen der allgemeinen Formel (1) in einem Lösungsmittel oder Lösungsmittelgemisch, wobei X in diesem Fall auch bis zu 100 Mol-% aus Protonen bestehen kann;
b) gegebenenfalls Neutralisation der erhaltenen Lösung oder Dispersion mittels einer oder mehrerer Basen, so dass mindestens 90 Mol-% der Sulfonsäure-Gruppierungen in die Salzform überführt werden,
c) Zugabe eines oder mehrerer Vernetzer mit mindestens zwei olefinischen Doppelbindungen zu der gemäß a) und b) erhaltenen Lösung oder Dispersion und
d) Starten der Polymerisation in an sich bekannter Weise, z.B. durch radikalbildende Verbindungen, bei einer Temperatur von 10 bis 150°C, wobei die Wahl des in a) erwähnten Lösungsmittels oder Lösungsmittelgemisches so erfolgt, dass die erhaltenen Polymerisate weitgehend im Lösungsmittel oder Lösungsmittelgemisch unlöslich sind.

In einer bevorzugten Ausführungsform wird die Polymerisation als Fällungspolymerisation durchgeführt.

Im einzelnen können die beschriebenen Polymere durch Fällungspolymerisation wie folgt hergestellt werden. Als Reaktionsmedium dient vorzugsweise ein wasserlöslicher Alkohol oder ein Gemisch mehrerer wasserlöslicher Alkohole mit 1 bis 4 C-Atomen, vorzugsweise tert.-Butanol, wobei der Wassergehalt des Alkohols oder Alkoholgemisches 10 Gew.-% nicht überschreiten darf. Die Wahl der Art und Menge des Lösungsmittels hat so zu erfolgen, dass die eingesetzte Menge an 2-Acrylamido-2-methylpropansulfonsäure und/oder deren Salz weitgehend löslich oder dispergierbar ist. Unter weitgehend löslich oder dispergierbar ist zu verstehen, dass sich auch nach Abstreifen des Rührwerks kein festes Material aus der Lösung oder Dispersion absetzt. Das im Verlaufe der Reaktion entstehende Polymerisat hingegen soll in dem gewählten Lösungsmittel (oder -gemisch) jedoch weitgehend unlöslich sein. Unter weitgehend unlöslich ist hierbei zu verstehen, dass im Verlauf der Polymerisation eine gut rührbare breiige Polymermasse entsteht, in der sich keine Klumpen oder Verklebungen bilden dürfen. Das durch Absaugen der Paste erhältliche Filtrat darf einen Feststoffgehalt von maximal 5 Gew.-% aufweisen. Sind die Polymerisate in stärkerem Ausmaß im gewählten Lösungsmittel oder Lösungsmittelgemisch löslich, kann es beim Trocknen der Polymerisatpaste zu Verklumpungen kommen.

Die Polymerisationsreaktion selbst wird in an sich bekannter Weise, z.B. durch radikalbildende Verbindungen wie Azoinitiatoren (z.B. Azo-bis-isobutyronitril), Peroxiden (z.B. Dilaurylperoxid) oder Persulfaten in einem geeigneten Temperaturintervall von 20 bis 120°C, vorzugsweise zwischen 40 und 80°C, ausgelöst und über einen Zeitraum von 30 Minuten bis mehreren Stunden fortgeführt.

Die nach dem erfindungsgemäßen Verfahren hergestellten Polymerkonzentrate enthalten neben dem Polymer noch einen oder mehrere Emulgatoren und/oder ein Lösungsmittel oder Lösungsmittelgemisch in der angegebenen Menge. Bei Verwendung von Emulgatoren als alleiniger Komponente II) ist der Anteil des Lösungsmittels bzw. Lösungsmittelgemischs somit 0 % und entsprechend ist der Anteil der Emulgatoren 0 %, wenn die Komponente II) nur aus einem Lösungsmittel bzw. Lösungsmittelgemisch besteht. Bevorzugt verwendet man als zweite Komponente eine Mischung aus Emulgator und Lösungsmittel bzw. Lösungsmittelgemisch.

Als Emulgatoren kommen in Betracht Anlagerungsprodukte von 0 bis 30 Mol Alkylenoxid, insbesondere Ethylen-, Propylen-, Butylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitanester; (C₁₂-C₁₈)-Fettsäuremonound -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Bevorzugt sind flüssige Fettsäureester, die sowohl ethoxyliert (PEG-10 Polyglyceryl-2 Laurate) als auch nicht ethoxyliert (Polyglyceryl-2 Sesquiisostearate) sein können.

Weitere nach dem erfindungsgemäßen Verfahren hergestellte Dispersionskonzentrate enthalten bevorzugt Sorbitolester, hergestellt durch Reaktion von Sorbitol mit Fettsäuremethylestern oder Fettsäuretriglyceriden. Der Fettsäurerest in den Fettsäuremethylestern und Fettsäuretriglyceriden enthält im allgemeinen 8 bis 22 C-Atome und kann geradkettig oder verzweigt, gesättigt oder ungesättigt sein. Beispiele hierfür sind Palmitinsäure, Stearinsäure, Laurinsäure, Linolsäure, Linolensäure, Isostearinsäure oder Ölsäure. Als Fettsäuretriglyceride kommen alle nativen tierischen oder pflanzlichen Öle, Fette und Wachse in Frage, beispielsweise Olivenöl, Rapsöl, Palmkernöl, Sonnenblumenöl, Kokosöl, Leinöl, Ricinusöl, Sojabohnenöl, gegebenenfalls auch in raffinierter oder hydrierter Form. Da diese natürlichen Fette, Öle und Wachse normalerweise Mischungen von Fettsäuren mit unterschiedlicher Kettenlänge darstellen, gilt dies auch für die Fettsäurereste in den erfindungsgemäß eingesetzten Sorbitolestern. Die erfindungsgemäß eingesetzten Sorbitolester können auch alkoxyliert sein, vorzugsweise ethoxyliert.

Des weiteren können anionische Emulgatoren, wie ethoxylierte und nicht ethoxylierte mono-, di- oder tri-Phosphorsäureester, aber auch kationische Emulgatoren wie mono-, di- und tri-Alkylquats und deren polymere Derivate eingesetzt werden.

Ebenfalls geeignet sind Gemische von Verbindungen aus mehreren dieser Substanzklassen.

Die nach dem erfindungsgemäßen Verfahren hergestellten Konzentrate können neben dem Polymer auf Basis von Acryloyldimethyltaurinsäure bzw. deren Salzen ein oder mehrere Lösungsmittel enthalten, bevorzugt aus der Gruppe der Kohlenwasserstoffe, Esteröle, pflanzlichen Öle und Silikonöle.

Die erfindungsgemäß eingesetzten Lösungsmittel umfassen Öle wie Kohlenwasserstofföle mit linearen oder verzweigten, gesättigten oder ungesättigten C₇-C₄₀-Kohlenstoffketten, beispielsweise Vaseline, Dodecan, Isododecan, Cholesterol, Lanolin, hydrierte Polyisobutylene, Docosane, Hexadecan, Isohexadecan, Paraffine und Isoparaffine;

Öle pflanzlichen Ursprungs, insbesondere flüssige Triglyceride wie Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Olivenöl, Erdnussöl, Rapsöl und Kokosnussöl; Öle tierischen Ursprungs, beispielweise Rindertalg, Perhydrosqualen, Lanolin; synthetische Öle wie Purcellinöl, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Alkoholester von C₁-C₁₀-Carbonsäuren oder C₂-C₃₀-Dicarbonsäuren, C₁-C₃₀-Carbonsäuremonoester und Polyester von Zucker, C₁-C₃₀-Monoester und Polyester von Glycerin; Wachse wie Bienenwachs, Paraffinwachs oder Mikrowachse, gegebenenfalls in Kombination mit hydrophilen Wachsen, wie z.B. Cetylstearylalkohol; fluorierte und perfluorierte Öle; Monoglyceride von C₁-C₃₀-Carbonsäuren, Diglyceride von C₁-C₃₀-Carbonsäuren, Triglyceride von C₁-C₃₀-Carbonsäuren, beispielsweise Triglyceride der Capryl/Caprinsäuren, Ethylenglykolmonoester von C₁-C₃₀-Carbonsäuren, Ethylenglycoldiester von C₁-C₃₀-Carbonsäuren, Propylenglycolmonoester von C₁-C₃₀-Carbonsäuren, Propylenglycoldiester von C₁-C₃₀-Carbonsäuren, sowie poropoxilierte und ethoxilierte Derivate der oben genannten Verbindungsklassen.

An Silikonölen in Betracht kommen Dimethylpolysiloxane, Cyclomethicone, Polydialkylsiloxane R₃SiO(R₂SiO)ₓSiR₃, worin R für Methyl oder Ethyl, besonders bevorzugt für Methyl, steht und x eine Zahl von 2 bis 500 ist, beispielsweise die unter den Handelsnamen VICASIL (General Electric Company), DOW CORNING 200, DOW CORNING 225, DOW CORNING 200 (Dow Corning Corporation) erhältlichen Dimethicone; Trimethylsiloxysilicate [(CH₂)₃SiO)_{1/2}]ₓ[SiO₂]_{y}, wobei x für eine Zahl von 1 bis 500 und y für eine Zahl von 1 bis 500 steht; Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoyx-, fluor- und/oder alkylmodifizierte Silikonverbindungen, sowie Polyethersiloxan-Copolymere, wie in US 5,104,645 und den darin zitierten Schriften beschrieben, die bei Raumtemperatur sowohl flüssig als auch harzförmig vorliegen können.

In einer bevorzugten Ausführungsform bestehen die nach dem erfindungsgemässen Verfahren hergestellten Konzentrate aus Dispersionen der Polymere in einer flüssigen Matrix enthaltend Öl, Emulgator, Dispergator und/oder Wasser. Weiterhin bevorzugt sind dabei flüssig-disperse Formen mit möglichst hohem Polymeranteil und niedriger Viskosität bei gleichzeitig hoher Stabilität der Dispersion. Bevorzugt werden in den erfindungsgemäßen Konzentraten Öle, Emulgatoren bzw. Dispergatoren eingesetzt, die in kosmetischen, pharmazeutischen und dermatologischen Formulierungen akzeptabel sind.

Die nach dem erfindungsgemäßen Verfahren hergestellten Konzentrate eignen sich als Verdicker, Konsistenzgeber, Emulgator, Solubilisator, Dispergator, Gleitmittel, Haftmittel, Conditioner und/oder Stabilisator hervorragend zur Formulierung von kosmetischen, pharmazeutischen und dermatologischen Mitteln, insbesondere von Öl-in-Wasser Emulsionen in Form von Cremes, Lotionen, Reinigungsmilch, Cremegele, Sprühemulsionen, z.B. Bodylotions, After-Sun Lotions, Sonnenschutzmittel und Deo-Sprays.

Die Polymerkonzentrate werden in den kosmetischen, pharmazeutischen und dermatologischen Zubereitungen in Gewichtsmengen von 0,01 bis 10 Gew.-%, bevorzugt 0,1 bis 5 Gew.-%, besonders bevorzugt 0,5 bis 3 Gew.-%, bezogen auf die fertigen Mittel eingesetzt.
Die kosmetischen, pharmazeutischen und dermatologischen Zubereitungen können anionische, kationische, nichtionische, zwitter-ionische und/oder amphotere Tenside, sowie weitere Hilfs- und Zusatzstoffe, kationische Polymere, Filmbildner, Überfettungsmittel, Stabilisatoren, biogene Wirkstoffe, Glycerin, Konservierungsmittel, Perlglanzmittel, Farb- und Duftstoffe, Lösungsmittel, Trübungsmittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden.

Zusätzlich können die kometischen, pharmazeutischen und dermatologischen Zubereitungen organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, tert.-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol. Hydrotrop wirken kurzkettige Aniontenside, insbesondere Arylsulfonate, beispielsweise Cumol- oder Toluolsulfonat.

Die nachfolgenden Beispiele von Konzentraten mit Polymeren auf Basis von Acryloyldimethyltaurinsäure bzw. deren Salzen sollen den Gegenstand der Erfindung näher erläutern, ohne ihn darauf einzuschränken. Bei den Prozentangaben handelt es sich um Gew.-%.

### Beispiel A (aus Tabelle) (hohe Emulgatorkonzentration)

In einem 1-Liter-Planschliffkloben mit Temperaturfühler, Rückflusskühler, KPG-Rührer und pH-Kontrolle werden 500 g tert.-Butanol und 80 g Acryloyldimethyltaurinsäure bei 30°C vorgelegt. Anschließend wird durch Einleitung von gasförmigem Ammoniak neutralisiert und 1,9 g Trimethylolpropantriacrylat (Vernetzer) zu der Reaktionsmischung zugegeben. Die Reaktionsmischung wird anschließend durch Einleiten von N₂ inertisiert, auf 60°C aufgeheizt und die Reaktion nach 30 Minuten durch Zugabe von 1,2 g Dilauroylperoxid gestartet. Es kommt zu einer exothermen Reaktion, bei der die Innentemperatur um mehrere Grad ansteigt. Nach etwa 10 Minuten kommt es zur Ausfällung des entstehenden Polymers, was sich in einem ständigen Anstieg der Lösungsviskosität bemerkbar macht. Nach Beendigung der exothermen Phase (etwa 20-30 Minuten) wird die Reaktionsmischung zur Siedehitze erhitzt und 2 Stunden zur Vervollständigung der Reaktion nachgekocht. Dabei sinkt die Viskosität der Lösung wieder ab. Danach wird der Rückflusskühler durch eine Destillationsbrücke ersetzt. Nun werden 45 g Hostaphat KL 340D, 75 g Emulsogen SRO, 25 g Mineralöl (niedrig-viskos) und 25 g Isopropylpalmitat zu der Polymersuspension zugegeben und die Hauptmenge an tert.-Butanol anschließend unter Rühren destillativ entfernt. Durch Anlegen eines Vakuums werden die Reste des tert.-Butanols aus der Mischung entfernt. Es ist darauf zu achten, dass das angelegte Vakuum zwar die destillative Abtrennung des tert.-Butanols ermöglicht, die bei diesem Druck korrespondierende Siedetemperatur des Lösemittels allerdings nicht überschreitet. Nach erfolgter Abtrennung des tert.-Butanols wird abgekühlt und das Produkt aus dem Kolben ausgetragen.

### Beispiel C (aus Tabelle) (niedrige Emulgatorkonzentration)

In einem 1-Liter-Planschliffkloben mit Temperaturfühler, Rückflusskühler, KPG-Rührer und pH-Kontrolle werden 400 g tert.-Butanol und 80 g Acryloyldimethyltaurinsäure bei 30°C vorgelegt. Anschließend wird durch Einleitung von gasförmigem Ammoniak neutralisiert und 1,65 g TMPTA (Trimethylolpropantriacrylat) zu der Reaktionsmischung zugegeben. Die Reaktionsmischung wird anschließend durch Einleiten von N₂ inertisiert, auf 60°C aufgeheizt und die Reaktion nach 30 Minuten durch Zugabe von 1,5 g Dilauroylperoxid gestartet. Es kommt zu einer exothermen Reaktion, bei der die Innentemperatur um mehrere Grad ansteigt. Nach etwa 10 Minuten kommt es zur Ausfällung des entstehenden Polymers, was sich in einem ständigen Anstieg der Lösungsviskosität bemerkbar macht. Nach Beendigung der exothermen Phase (etwa 20-30 Minuten) wird die Reaktionsmischung zur Siedehitze erhitzt und 2 Stunden zur Vervollständigung der Reaktion nachgekocht. Dabei sinkt die Viskosität der Lösung wieder ab. Danach wird der Rückflusskühler durch eine Destillationsbrücke ersetzt. Nun werden 6 g Hostacerin DGI, 4 g Hostaphat KL 340D, 55 g Mineralöl (niedrig-viskos) und 55 g Isopropylpalmitat zu der Polymersuspension hinzugegeben und anschließend die Hauptmenge an tert.-Butanol unter Rühren destillativ entfernt. Durch Anlegen eines Vakuums werden die Reste des tert.-Butanols aus der Mischung entfernt. Es ist darauf zu achten, dass das angelegte Vakuum zwar die destillative Abtrennung des tert.-Butanols ermöglicht, die bei diesem Druck korrespondierende Siedetemperatur des Lösemittels allerdings nicht überschreitet.

Nach erfolgter Abtrennung des tert.-Butanols wird abgekühlt und das Produkt aus dem Kolben ausgetragen.

Es wurden verschiedene Basisrezepturen mit unterschiedlichen Emulgator- und Ölkonzentrationen hergestellt. Die resultierenden Polymerkonzentrate wurden nach Aussehen, Viskosität sowie Stabilität (Sedimentierung bei Lagerung 25°C 3 Wochen) beurteilt. Tabelle 1 zeigt Beispiele von Konzentraten, die fließfähig und lagerstabil sind.

**Tabelle 1**

| Dispersionskonzentrat | A | B | C | D |
|---|---|---|---|---|
| Polymer | 32 | 36 | 40 | 30 |
| Hostacerin DGI | - | 30 | 3 | 51 |
| Hostaphat KL 340 D | 18 | 18 | 2 | 13 |
| Emulsogen SRO | 30 | - | - | - |
| Mineral Öl, niedrig viskos | 10 | - | 27,5 | 6 |
| Isopropyl Palmitate | 10 | - | 27,5 | - |
| Myritol 318 | - | 16 | - | - |

Die Zahlenangaben in Tabelle 1 bedeuten Gew.-%. Die Dispersionskonzentrate B und D wurden analog zu A hergestellt, wobei jedoch die Emulgatoren und Öle variiert wurden.

| | |
|---|---|
| Hostacerin DGI | Polyglyceryl-2-Sesquiisostearate |
| Hostaphat KL 340 D | Trilaureth-4 Phosphate |
| Emulsogen SRO | Rapeseed Oil Sorbitol Esters |
| Myritol 318 | Caprylic/Capric Triglyceride |

Beispiele für kosmetische Präparate auf der Basis der erfindungsgemäßen Konzentrate. Die Prozentangaben bedeuten Gew.-%.

### Beispiel 1: Feuchtigkeitsspendende Lotion

| | | |
|---|---|---|
| A | Almondöl | 7,00 % |
| | Cyclomethicone | 5,00 % |
| B | Dispersionskonzentrat A | 4,00 % |
| C | Glycerin | 7,00 % |
| | Wasser | ad 100 % |
| | Konservierungsmittel | q.s. |
| D | Duftstoff | 0,30 % |

### Herstellung

I A und B mischen.
II Lösung von C in einrühren.
III D zu II geben.
IV homogenisieren
V pH 5,5

### Beispiel 2: Sonnenschutzlotion

| | | |
|---|---|---|
| A | Vaselin | 5,00% |
| | Paraffinöl | 10,00% |
| | Dispersionskonzentrat B | 2,00 % |
| | Tocopherylacetat | 1,00 % |
| | Octylmethoxycinnamat | 2,00 % |
| | Parasol 1789 | 0,20 % |
| B | Ethanol | 10,00 % |
| C | Butylenglykol | 5,00 % |
| | Wasser | ad 100 % |

### Herstellung

I A und C werden getrennt auf 75°C erwärmt, danach vereinigt und unter Rühren auf 65°C abgekühlt, homogenisiert und weiter auf 35°C abgekühlt,
II B wird in I eingerührt, es wird homogenisiert und auf Raumtemperatur abgekühlt

### Beispiel 3: O/W - Hautmilch

| | | |
|---|---|---|
| A | Isopropylpalmitat | 4,00 % |
| | Mandelöl 5,00 % | 4,00 % |
| | Weizenkeimöl | 1,00 % |
| | Cetearylisononanoat | 8,00 % |
| | ® Cetiol SN (Henkel) | |
| B | Dispersionskonzentrat C | 1,50 % |
| C | Wasser | ad 100 % |
| D | Duftstoffe | 0,30 % |

### Herstellung

I B unter Rühren zu A hinzugeben ,
II C und D zu I hinzurühren
III Emulsion homogenisieren

## Patentansprüche

1. Verfahren zur Herstellung von Konzentraten in flüssiger oder flüssig-disperser Form, enthaltend
I) 5 bis 80 Gew.-% eines Polymers, das in statistischer Verteilung zu 90 bis 99,99 Gew.-% aus Monomeren der allgemeinen Formel (1)
H₂C=CHCONHC(CH₃)₂CH₂SO₃X (1)
worin X für ein Kation oder ein Gemisch von Kationen steht und X nicht zu mehr als 10 Mol-% aus Protonen besteht, und
zu 0,01 bis 10 Gew.-% aus Monomeren mit mindestens zwei olefinischen Doppelbindungen hervorgegangen ist,
II) 20 bis 95 Gew.-% eines oder mehrerer Emulgatoren und/oder eines Lösungsmittels oder Lösungsmittelgemischs und
III) 0 bis 30 Gew.-% Wasser,
**dadurch gekennzeichnet, dass** das Konzentrat hergestellt wird durch
a) radikalische Polymerisation der Monomere gemäß Formel (1) in Gegenwart der Monomere mit mindestens zwei Doppelbindungen in einem Polymerisationsmedium, das sich bezüglich radikalischer Polymerisationsreaktionen weitestgehend inert verhält und die Bildung hoher Molekulargewichte zulässt,
b) dem Gemisch aus Polymer und Polymerisationsmedium ein höhersiedendes Lösungsmittel bzw. Lösungsmittelgemisch und/oder ein oder mehrere Emulgatoren und gegebenenfalls Wasser zugesetzt wird, wobei der Siedepunkt des höhersiedenden Lösungsmittels bzw. Lösungsmittelgemisches mindestens 10°C höher liegt als der des zur Polymerisation eingesetzten Polymerisationsmediums
und
c) das niedriger siedende Polymerisationsmedium, gegebenenfalls bei einem Druck, der im Vergleich zu Atmosphärendruck erniedrigt ist, und gegebenenfalls bei einer Temperatur, die im Vergleich zu Raumtemperatur erhöht ist, entfernt wird.

2. Verfahren nach Anspruch 1, **dadurch gekennzeichnet, dass** das Gegenion X in der Formel (1) für ein Proton, ein Kation eines Alkalimetalls, ein Äquivalent eines Kations eines Erdalkalimetalls oder für ein Ammoniumion steht.

3. Verfahren nach Anspruch 1 oder 2, **dadurch gekennzeichnet, dass** die Monomere mit mindestens zwei olefinischen Doppelbindungen ausgewählt sind aus Dipropylglykoldiällylether, Polyglykoldiallylether, Triethylenglykol-divinylether, Hydrochinondiallylether, Tetraallyloxyethan oder anderen Allyl- oder Vinylethern multifunktioneller Alkohole, Tetraethylenglykoldiacrylat, Triallylamin, Trimethylolpropandiallylether, Methylen-bis-acrylamid, Divinylbenzol oder Trimethylolpropyltri(meth)acrylat.

4. Verfahren nach einem oder mehreren der Ansprüche 1 bis 3, **dadurch gekennzeichnet, dass** das Polymerisationsmedium ausgewählt ist aus Wasser und niederen, tertiären Alkoholen oder Kohlenwasserstoffen mit 3 bis 30 C-Atomen.

5. Verfahren nach Anspruch 4, **dadurch gekennzeichnet, dass** das Polymerisationsmedium tert.-Butanol ist.

## Claims

1. A process for the preparation of concentrates in liquid or liquid-disperse form comprising
I) 5 to 80% by weight of a polymer which, in random distribution, has come, in an amount of 90 to 99.99% by weight, from monomers of the formula (1)
H₂C=CHCONHC (CH₃)₂CH₂SO₃X (1)
in which X is a cation or a mixture of cations, and X consists of not more than 10 mol% of protons, and,
in an amount of 0.01 to 10% by weight, from monomers with at least two olefinic double bonds,
II) 20 to 95% by weight of one or more emulsifiers and/or a solvent or solvent mixture, and
III) 0 to 30% by weight of water,
wherein the concentrate is prepared by
a) free-radical polymerization of the monomers of formula (1) in the presence of the monomers having at least two olefinic double bonds, in a polymerization medium which behaves largely inertly with regard to free-radical polymerization reactions and permits the formation of high molecular weights,
b) a higher-boiling solvent or solvent mixture and/or one or more emulsifiers and optionally water is added to the mixture of polymer and polymerization medium, where the boiling point of the higher-boiling solvent or solvent mixture is at least 10°C higher than that of the polymerization medium used for the polymerization and
c) the lower-boiling polymerization medium is removed, optionally at a pressure which is lowered relative to atmospheric pressure, and optionally at a temperature which is increased relative to room temperature.

2. The process as claimed in claim 1, wherein the counterion X in the formula (1) is a proton, a cation of an alkali metal, an equivalent of a cation of an alkaline earth metal or is an ammonium ion.

3. The process as claimed in claim 1 or 2, wherein the monomers with at least two olefinic double bonds are selected from dipropyl glycol diallyl ether, polyglycol diallyl ether, triethylene glycol divinyl ether, hydroquinone diallyl ether, tetraallyloxyethane or other allyl or vinyl ethers of multifunctional alcohols, tetraethylene glycol diacrylate, triallylamine, trimethylolpropane diallyl ether, methylenebisacrylamide, divinylbenzene or trimethylolpropyl tri(meth)acrylate.

4. The process as claimed in one or more of claims 1 to 3, wherein the polymerization medium is selected from water and lower, tertiary alcohols or hydrocarbons having 3 to 30 carbon atoms.

5. The process as claimed in claim 4, wherein the polymerization medium is tert-butanol.

## Revendications

1. Procédé de préparation de concentrés sous forme liquide ou en dispersion dans un liquide, contenant
I) 5 à 80 % en poids d'un polymère qui en répartition statistique est constitué de 90 à 99,99 % en poids de monomères de formule générale (1)
H₂C=CHCONHC (CH₃)₂CH₂SO₃X (1)
dans laquelle X est un cation ou un mélange de cations et X est constitué de protons en une quantité non supérieure à 10 % en moles, et a été obtenu, en une quantité de 0,01 à 10 % en poids, à partir de monomères ayant au moins deux doubles liaisons oléfiniques,
II) 20 à 95 % en poids d'un ou plusieurs émulsifiants et/ou d'un solvant ou d'un mélange de solvants, et
III) 0 à 30 % en poids d'eau,
**caractérisé en ce que** le concentré est préparé comme suit:
a) polymérisation radicalaire des monomères de formule (1), en présence des monomères ayant au moins deux doubles liaisons oléfiniques, dans un milieu de polymérisation qui vis-à-vis de réactions de polymérisation radicalaire a un comportement essentiellement inerte, et autorise la formation de masses moléculaires élevées,
b) on ajoute au mélange du polymère et du milieu de polymérisation un solvant ou un mélange de solvants à haut point d'ébullition et/ou un ou plusieurs émulsifiants et éventuellement de l'eau, le point d'ébullition du solvant ou du mélange de solvants à haut point d'ébullition étant d'au moins 10°C supérieur à celui du milieu de polymérisation utilisé pour la polymérisation, et
c) on élimine le milieu de polymérisation à bas point d'ébullition, éventuellement sous une pression qui est inférieure à la pression atmosphérique, et éventuellement à une température qui est supérieure à la température ambiante.

2. Procédé selon la revendication 1, **caractérisé en ce que** le contre-ion X, dans la formule (1), représente un proton, un cation d'un métal alcalin, un équivalent d'un cation d'un métal alcalin ou un ion ammonium.

3. Procédé selon la revendication 1 ou 2, **caractérisé en ce que** les monomères ayant au moins deux doubles liaisons oléfiniques sont choisis parmi l'éther diallylique du dipropylglycol, l'éther diallylique du polyglycol, l'éther divinylique du triéthylèneglycol, l'éther diallylique de l'hydroquinone, le tétraallyloxyéthane ou d'autres éthers allyliques ou vinyliques de polyalcools, le diacrylate de tétraéthylèneglycol, la triallylamine, l'éther diallylique du triméthylolpropane, le méthylènebisacrylamide, le divinylbenzène ou le tri(méth)acrylate de triméthylolpropyle.

4. Procédé selon l'une ou plusieurs des revendications 1 à 3, **caractérisé en ce que** le milieu de polymérisation est choisi parmi l'eau et les alcools tertiaires inférieurs ou les hydrocarbures ayant 3 à 30 atomes de carbone.

5. Procédé selon la revendication 4, **caractérisé en ce que** le milieu de polymérisation est le tert-butanol.
